# EUROPÄISCHE PATENTSCHRIFT

(11) **EP 1 273 320 B1**
(45) Veröffentlichungstag und Bekanntmachung des Hinweises auf die Patenterteilung: **27.04.2005**
(21) Anmeldenummer: 01114823.6
(22) Anmeldetag: 28.06.2001
(51) Int. Cl.: A61N 2/02, A61N 2/00, A61B 5/055

(54) **Vorrichtung für transcraniale magnetische Stimulation**
Apparatus for transcranial magnetic stimulation
Appareil de stimulation magnétique transcranienne

(43) Veröffentlichungstag der Anmeldung: 08.01.2003
(73) Patentinhaber: BrainLAB AG, 85551 Kirchheim/Heimstetten (DE)
(72) Erfinder: Tanner, Philipp Dr., 80769 München (DE); Hartlep, Andreas Dr., 80803 München (DE); Wist, Henrik, 80796 München (DE); Wendicke, Kerstin, 81476 München (DE); Weyh, Thomas Dr., 80803 München (DE)
(74) Vertreter: Schwabe - Sandmair - Marx

(56) Entgegenhaltungen:
- WO-A-98/06342
- US-A- 5 644 234
- US-A- 5 738 625
- T. KRINGS B.R. BUCHBINDER ET AL.: "Stereotactic Transcranial Magnetic Stimulation: Correlation with Direct Electrical Cortical Stimulation" NEUROSURGERY, Bd. 41, Nr. 6, Dezember 1997 (1997-12), Seiten 1319-1326, XP001039743 Boston, Massachusetts
- ETTINGER ET AL: 'Non-invasive Functional Brain Mapping Using Registered Transcranial Magnetic Stimulation' IEEE

## Beschreibung

Die vorliegende Erfindung bezieht sich auf eine Vorrichtung für eine transcraniale magnetische Stimulation, insbesondere zur nicht-invasiven Lokalisation bestimmter Bereiche eines Gehirns, wie zum Beispiel sogenannter primärer Hirnareale. Damit können zum Beispiel die Hirnfunktionen kartiert, also bestimmten Hirnbereichen zugeordnet werden.

In verschiedenen medizinischen Bereichen, wie zum Beispiel der Neurologie, der Psychiatrie oder der Gehirn-Chirurgie ist es wünschenswert bestimmte funktionale Bereiche des Gehirns lokalisieren zu können, um Hirnfunktionen zu kartieren. Soll zum Beispiel durch einen chirurgischen Eingriff ein Hirntumor entfernt werden, so sollte der Tumor soweit wie möglich entfernt werden, wobei jedoch sogenannte primäre Hirnareale, welche bezüglich der Motorik, Sensorik, der Sprache oder den visuellen Fähigkeiten einer Person eine entscheidende Rolle spielen, nach Möglichkeit nicht verletzt werden sollen. Diese Bereiche sollten bei einem chirurgischen Eingriff wenn möglich gar nicht oder nur in äußerst geringem Umfang verletzt werden.

Das Auffinden solcher besonderen Hirnbereiche wurde nach einem bekannten direkten Verfahren intra-operativ durchgeführt, wobei mittels Elektroden eine direkte cortikale Stimulation (DCS) an einem geöffneten Schädel erfolgte. Dabei wurde eine Elektrode in einen bestimmten Bereich des Gehirns eingebracht und ein elektrischer Impuls angelegt, wobei die auf den elektrischen Impuls folgende Reaktion der untersuchten Person, zum Beispiel das Zucken eines Muskels oder das Wahrnehmen von visuellen Eindrücken beobachtet wurde. Die durch die direkte cortikale Stimulation aufgefundenen besonderen Hirnbereiche wurden mittels aufgelegter kleiner Plättchen markiert, welche für einen Chirurgen bei einer nachfolgenden Gehirnoperation eine Orientierungshilfe bezüglich der möglichst nicht zu verletzenden Himbereiche darstellten. Die direkte cortikale Stimulation stellt bis zum heutigen Tag das präziseste Verfahren zur Kartierung von Hirnfunktionen dar und ermöglicht eine Genauigkeit bei der Auffindung bestimmter Hirnareale im Bereich von wenigen Millimetern. Jedoch kann dieses Verfahren nur intra-operativ durchgeführt werden, wobei die untersuchte Person bei vollem Bewusstsein sein muss. Dies kann bei der Anwendung dieses Verfahrens jedoch zu Problemen führen, da dieser Zustand für die untersuchte Person unangenehm ist und die Person, falls es zu Komplikationen kommen sollte, aufgrund der geöffneten Schädeldecke nicht einfach hingelegt und ruhig gestellt werden kann.

Es sind weiterhin verschiedene indirekte Verfahren zur Kartierung von Hirnfunktionen bekannt, mit welchen jedoch nur eine erheblich geringere Genauigkeit beim Auffinden spezifischer Hirnareale erreicht werden kann. So muss zum Beispiel bei der funktionellen Kernspintomographie (fMRI) eine untersuchte Person bestimmte Aktionen wie zum Beispiel eine Handbewegung ausführen, welche die Durchblutung der diesen Aktionen zugeordneten Hirnbereichen fördert. Diese Veränderung der Durchblutung bestimmter Himbereiche kann aufgrund der Entkopplung von Durchblutung und Sauerstoffverbrauch während der neuronalen Aktivität gemessen werden, da hierdurch eine Hyperoxygenierung und damit ein Abfall der Konzentration des paramagnetischen Deoxy-Hämoglobins (BOLD-Effekt) auftritt, was dann als sogenanntes "endogenes Kontrastmittel" mittels geeigneter Sequenzen mit Kernspintomographie gemessen werden kann. Jedoch ist dieses Verfahren wie oben erwähnt, relativ ungenau und liefert nur eine räumliche Auflösung im Bereich von etwa 0,5 bis 1,0 cm.

Aus Neurosurgery 1992-1998, December 1997, Volume 41, Number 6, 1319 "Stereotactic Transcranial Magnetic Stimulation: Correlation with Direct Electrical Cortical Stimulation" ist ein Verfahren bekannt, bei welchem eine stereotaktische transcraniale magnetische Stimulation (TMS) für das präoperative funktionale Mapping des motorischen Cortex verwendet wird. Der Kopf eines Patienten wird dabei fest und unbeweglich mit einer Kopflehne verbunden, wobei eine Schwenkarm vorgesehen ist, an welchem eine 8-förmige Spule so angebracht ist, dass die Armspitze unter dem Spulenschnittpunkt liegt. Der Arm wird dabei so ausgerichtet, dass die unter dem Schnittpunkt der beiden Spulen liegenden Spitze auf einen bestimmten Bereich zeigt, in welchem ein Strom induziert werden soll.

Aus der WO 98/06342 ist eine Vorrichtung zur Stimulation bestimmter Bereiche eines Gehirns mit einer Induktionsvorrichtung bekannt, welche einen Center Port aufweist, um die Stelle, an welcher die Stimulationsvorrichtung auf dem Kopf angeordnet ist, präzise zu markieren.

Aus IEEE, Proceedings of MMBIA, 1996, p. 32-41 "Non invasive Functional Brain Mapping Using Registered Transcranial Magnetic Stimulation" ist eine Vorrichtung bekannt, wobei Kernspinresonanzdaten segmentiert werden, um die Hautoberfläche zum Registrieren und die internen Strukturen zum Visualisieren zu vermenden, und wobei ein 3D-Modell eines Patienten zum Visualisieren und Identifizieren von internen Strukturen schalten werden kann.

Es ist eine Aufgabe der vorliegenden Erfindung eine Vorrichtung zur Stimulation bestimmter Bereiche eines Gehirns vorzuschlagen, mit welchen die Anwendung vereinfacht und die räumliche Genauigkeit der Stimulation und damit der Lokalisation bestimmter Hirnbereiche verbessert werden kann.

Diese Aufgabe wird durch eine Vorrichtung gemäß Anspruch 1 gelöst. Vorteilhafte Ausführungsformen ergeben sich aus den Unteransprüchen.

Bei einem Verfahren zur Stimulation bestimmter Bereiche eines Gehirns, um zum Beispiel eine nicht-invasive Lokalisation von primären Himarealen vornehmen zu können, wird die räumliche Struktur des Gehirns zum Beispiel mittels eines Kernspinresonanzverfahrens (MRI), einer Computertomographie (CT) oder einem anderen geeigneten Verfahren aufgenommen. Eine Induktionsvorrichtung, welche zum Erzeugen von für die Stimulation notwendigen Magnetfeldern verwendet wird, wird erfindungsgemäß vor Beginn der Stimulation untersucht. Dabei wird zum Beispiel berechnet, welches Magnetfeld von der Induktionsvorrichtung bei bestimmten hindurchfließenden Strömen erzeugt wird, insbesondere wo das von der Induktionsvorrichtung erzeugte magnetische Feld am größten ist, also zum Beispiel die größte Feldstärke aufweist. Hierzu kann zum Beispiel eine optische genaue Untersuchung der Spulenwicklung bzw. Spulenwicklungen der Induktionsvorrichtung vorgenommen werden, wobei aus diesen Spulenwicklungen der Bereich bzw. Punkt des maximalen durch die Induktionsvorrichtung erzeugbaren Magnetfeldes berechnet werden kann. Die Induktionsvorrichtung kann zum Beispiel auch geröntgt oder durch andere geeignete Verfahren untersucht werden, um eine genaue Analyse der Stromführung der Induktionsvorrichtung durchzuführen. Alternativ oder ergänzend zur Berechnung des durch die Induktionsvorrichtung erzeugbaren Magnetfeldes kann auch eine Messung vorgenommen werden, mit welcher der räumliche Bereich bzw. Punkt des größten durch die Induktionsvorrichtung erzeugbaren Magnetfeldes in Relation zur Induktionsvorrichtung ermittelt werden kann. Allgemein soll erfindungsgemäß eine Induktionsvorrichtung vor der Verwendung zur Stimulation bzw. Simulation zur Stimulation bestimmter Bereiche eines Gehirns untersucht bzw. analysiert werden, um zu ermitteln, in welchem räumlichen Lageverhältnis zur Induktionsvorrichtung der Bereich bzw. Punkt liegt, an welchem durch die Spule das maximale magnetische bzw. durch das magnetische Feld das maximal induzierte elektrische Feld erzeugt wird. Unter Verwendung dieses Untersuchungsergebnisses kann ein Simulationsmodell der Induktionsvorrichtung erstellt werden, so dass diese relativ zum zu untersuchenden Gehirn so angeordnet werden kann, dass ein gewünschter Bereich des Gehirns durch einen in der Induktionsvorrichtung fließenden Strom stimuliert werden kann. Es kann somit ein räumlich möglichst eng begrenztes starkes Magnetfeld auf einen kleinen Bereich des Gehirns fokussiert werden.

Ein Simulationsmodell des zu untersuchenden Kopfes kann erzeugt werden, wobei die aus der Aufnahme der räumlichen Struktur des Kopfes ermittelten Daten zur Verbesserung des Simulationsmodells verwendet werden können. Dadurch kann das durch die Induktionsvorrichtung erzeugte elektrische bzw. magnetische Feld durch geeignete Positionierung der Induktionsvorrichtung relativ genau auf einem bestimmten Punkt des Gehirns fokussiert werden, da die eine unterschiedliche Leitfähigkeit aufweisenden über dem Gehirn liegenden Schichten wie verschiedene Dielektrika wirken, die einen entscheidenden Einfluss auf die Fokussierung des Feldes auf dem Gehirn haben.

Die beiden oben beschriebenen Verfahren der Analyse und Simulation der Induktionsvorrichtung und der Modellierung des Kopfes können getrennt voneinander oder in Kombination eingesetzt werden, um die Fokussierung des durch die Induktionsvorrichtung erzeugten Feldes weiter zu verbessern.

Mit den oben beschriebenen Verfahren kann die transcraniale magnetische Stimulation (TMS) verbessert, insbesondere präzisiert werden, was eine präzise nicht-invasive Lokalisation bestimmter Gehirnbereiche, zum Beispiel der oben erwähnten primären Hirnareale ermöglicht, wodurch zum Beispiel die präoperative chirurgische Planung verbessert werden kann. Die Verfahren können jedoch auch zur Untersuchung oder Heilung anderer Funktionsstörungen des Gehirns eingesetzt werden, wie zum Beispiel zum Diagnostizieren einer Epilepsie oder der Diagnose der Parkinsonschen Krankheit. Unter Verwendung der Verfahren ist es möglich durch ein indirektes Verfahren eine genaue Kartierung von Hirnfunktionen vorzunehmen, ohne den Schädel einer Person öffnen zu müssen, um direkt auf das Gehirn zuzugreifen.

Vorteilhaft erfolgt die Modellierung des Kopfes durch Bildung eines finiten Mehr-Schalen-Modells, wobei zum Beispiel die Berechnung von elektrischen und/oder magnetischen Feldem mit Finite-Elemente-Methoden durchgeführt werden kann und die einzelnen Schalen vorteilhaft zum Beispiel als ineinanderliegende Kugel- oder Ellipsoidschalen mit einstellbarer Dicke modelliert werden können, welche zum Beispiel konzentrisch angeordnet sein können. Insbesondere ist eine Unterteilung in drei Schalen von Vorteil, wobei durch die jeweiligen Schalen von außen nach innen die Kopfhaut, die Schädelknochen und die Hirnoberfläche simuliert werden. Diese drei verschiedenen Kopfbereiche weisen unterschiedliche elektrische und magnetische Eigenschaften auf, so dass zum Beispiel eine Modellierung eines Kopfes durch drei Schalen mit zum Beispiel unterschiedlicher Dielektrizitätskonstante und/oder unterschiedlicher Leitfähigkeit recht gute Ergebnisse liefern kann, wenn die für die elektrischen bzw. magnetischen Parameter der einzelnen Schalen verwendeten Werte möglichst nahe an bei Versuchen ermittelten oder errechneten Werten liegen. Vorteilhaft wird das Schalenmodell noch in Abhängigkeit von einer durchgeführten Aufnahme der räumlichen Struktur eines zu untersuchenden Kopfes modifiziert, wobei zum Beispiel durch Streckung und/oder Verzerrung bzw. Verschiebung einzelner Schalen eine möglichst genaue Anpassung des Schalenmodells an die Geometrie eines zu untersuchenden Kopfes vorgenommen wird.

Bevorzugt werden an dem untersuchten Kopf und/oder an der Induktionsvorrichtung Marker, besonders bevorzugt passive Marker angebracht, um eine Tracking der Induktionsvorrichtung und/oder des Kopfes vornehmen zu können. Die Referenzierung und das Tracking von Personnen und/oder Instrumenten ist aus dem Stand der Technik bekannt und wird hier nicht näher beschrieben. Es wird ein Matching zwischen Induktionsvorrichtung und Kopf vorgenommen, um so die relative räumliche Lage zwischen Induktionsvorrichtung und Kopf möglichst präzise bestimmen zu können. Die Verwendung von Markern hat den Vorteil, dass eine untersuchte Person nicht ortsfest positioniert werden muss, sondern dass auch bei einer sich frei im Raum bewegenden Person eine präzise Stimulation bestimmter Hirnbereiche vorgenommen werden kann.

Als Induktionsvorrichtung kann zum Beispiel eine einzelne Spule bestehend aus einer oder mehreren Wicklungen oder eine Kombination aus Spulen verwendet werden. Besonders vorteilhaft wird eine Induktionsvorrichtung verwendet, bei welcher zwei in einer Ebene liegenden Spulen zueinander benachbart sind, so dass die Spulen in etwa die Gestalt einer "8" aufweisen.

Die Stimulation eines Hirnareals erfolgt bevorzugt durch an die Induktionsvorrichtung angelegte elektrische Impulse, welche eine Anstiegszeit im Bereich von einer Mikrosekunde bis einer Millisekunde und eine Dauer von 10 bis 1000 Mikrosekunden haben können. Dabei können die einzelnen Impulse mit einem periodischen Muster angelegt werden.

Bevorzugt erfolgt eine optische Anzeige der durch eine Aufnahme ermittelten räumlichen Struktur der Hirnoberfläche, vorteilhaft mit einer Anzeige des simulierten Stimulationsbereiches bei einer momentanen Lage der Induktionsvorrichtung. Unter Zuhilfenahme einer solchen optischen Anzeige kann eine Bedienperson zum Beispiel durch seitliches Kippen der Induktionsvorrichtung und/oder Bewegen der Induktionsvorrichtung auf den Kopf zu oder von dem Kopf weg die Position und Größe des Stimulationsbereiches verändern, wobei ein möglichst kleiner Fokussierpunkt angestrebt wird, um eine möglichst hohe räumliche Auflösung bei der Lokalisation bestimmter Hirnbereiche zu erhalten.

Vorteilhaft erfolgt die Positionierung der Induktionsvorrichtung relativ zu dem Kopf automatisch, wobei die Induktionsvorrichtung durch beispielsweise einen mit mehreren Freiheitsgraden bewegbaren Roboterarm so positioniert wird, dass eine Vielzahl von Punkten auf der Oberfläche des Gehirns mit einem möglichst kleinen Fokussierpunkt stimuliert werden. Hierzu kann der bewegliche Roboterarm fest an dem Kopf befestigt werden. Dabei können zum Beispiel von einer Mustererkennungssoftware geeignete Stimulationspunkte auf der Himoberfläche ausgewählt werden und/oder Stimulationspunkte von einer Bedienperson vorgegeben werden, welche dann von dem Roboterarm automatisch so angefahren werden, dass die positionierte Induktionsvorrichtung einen möglichst kleinen Bereich der Hirnoberfläche stimuliert. Zur automatischen Positionierung kann das Simulationsmodell der Induktionsvorrichtung und/oder des Kopfes verwendet werden.

Gemäß der Erfindung wird eine Vorrichtung zur Stimulation bestimmter Bereiche eines Gehirns vorgeschlagen, wobei eine Induktionsvorrichtung vorgesehen ist, welche fest mit Markern, bevorzugt passiven Markern verbunden ist. Eine solche mit Markern versehende Induktionsvorrichtung kann erfindungsgemäß einfach und präzise positioniert werden, um einen bestimmten Bereich des Gehirns mit möglichst kleinem Fokuspunkt zu stimulieren.

Erfindungsgemäß sind auch am zu untersuchenden Kopf Marker in fester räumlicher Anordnung angebracht, welche ebenso wie die mit der Induktionsvorrichtung verbundenen Marker durch eine Positionserfassungsvorrichtung erfasst werden können. Bevorzugt ist weiterhin eine Simulationsvorrichtung vorgesehen, mit welcher der durch die Induktionsvorrichtung zu stimulierende Stimulationsbereich im Gehirn bestimmt werden kann, wobei zur Simulation des Stimulationsbereiches wie oben beschrieben ein Modell der Induktionsvorrichtung und/oder des Kopfes verwendet werden kann.

Vorteilhaft ist eine Anzeigevorrichtung zur Anzeige der relativen Position zwischen Induktionsvorrichtung und Gehirn oder zur Anzeige des durch die Induktionsvorrichtung erzeugbaren Stimulationsfeldes auf dem Gehirn vorgesehen, welche es einer Bedienperson ermöglichen die Induktionsvorrichtung möglichst präzise relativ zum Gehirn zu positionieren, bevor ein Stimulationsimpuls ausgelöst wird.

Vorteilhaft weist die erfindungsgemäße Vorrichtung eine Vorrichtung zum automatischen Positionieren der Induktionsvorrichtung relativ zum Gehirn auf, wie zum Beispiel einen Roboterarm, welcher mit mehreren Freiheitsgraden bewegbar ist, also zum Beispiel ein oder mehrere Gelenke aufweist, so dass die Induktionsvorrichtung beispielsweise zum Kopf hin oder vom Kopf weg bewegt oder bezüglich der Kopfoberfläche gekippt werden kann, um so einen durch die Induktionsvorrichtung erzeugbaren Stimulationsbereich auf dem Gehirn möglichst klein zu machen.

Die Erfindung wird nachfolgend anhand bevorzugter Ausführungsbeispiele beschrieben werden. Es zeigen:
- Fig. 1: eine schematische Darstellung eines erfindungsgemäß verwendeten Drei-Schalen-Modells;
- Fig. 2a: eine schematische Darstellung eines Simulationsmodells nach dem Stand der Technik;
- Fig. 2b: eine schematische Darstellung des erfindungsgemäßen Simulationsmodells;
- Fig. 3: eine schematische Darstellung einer als Induktionsvorrichtung verwendbaren Spule;
- Fig. 4a: das durch eine einzelne Spule erzeugte magnetische Feld;
- Fig. 4b: das durch zwei benachbarte Spulen erzeugte magnetische Feld;
- Fig. 5: ein Prinzipschaubild der erfindungsgemäßen Vorrichtung zur Stimulation bestimmter Bereiche des Gehirns;
- Fig. 6: ein Ablaufdiagramm einer Ausführungsform des erfindungsgemäßen Verfahrens; und
- Fig. 7: eine Bildschirmanzeige des Gehirns mit simuliertem Stimulationsbereich zur Unterstützung der exakten Positionierung der Induktionsvorrichtung.

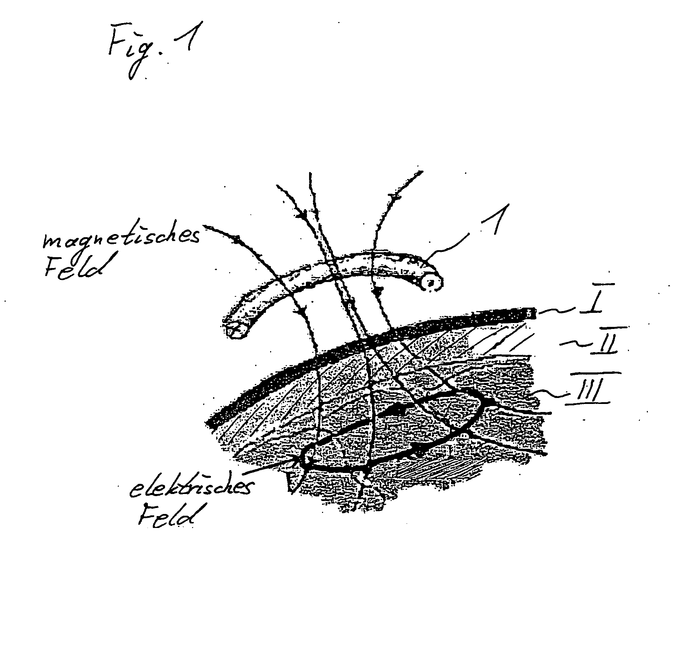

Fig. 1 zeigt schematisch ein erfindungsgemäßes Drei-Schalen-Modell eines Kopfes, wobei die äußerste Schale I die Kopfhaut modelliert, unter der durch die äußere Schale I modellierten Kopfhaut liegt die zweite Schale II, welche den Schädelknochen modelliert. Der Innenbereich des Drei-Schalen-Modells wird durch das Gehirn bzw. die Gehirnoberfläche III gebildet. Ein Strom in der durch eine Spule schematisch gezeigten Induktionsvorrichtung 1 verursacht das schematisch gezeigte magnetische Feld, welches von der Induktionsvorrichtung 1 ausgeht und durch die Kopfhaut und durch den Schädelknochen hindurchtritt, um im Gehirn, jeweils modelliert durch die drei Schalen I, II und III, ein schematisch gezeigtes elektrisches Feld zu induzieren, welches einen ringförmigen Stromfluss bewirkt, wodurch ein bestimmter Bereich des Gehirns stimuliert wird. Die Modellierung von Kopfhaut, Schädelknochen und Gehirn durch zwei äußere Schalen I und II, welche das modellierte Gehirn III umgeben, ermöglicht es, dass die Induktionsvorrichtung 1 an einer bestimmten Stelle in einem bestimmten Abstand vom Gehirn und bei einem bestimmten Kippwinkel relativ zur Oberfläche des Gehirns positioniert werden kann, um das durch das Magnetfeld der Induktionsvorrichtung auf der Hirnoberfläche induzierte elektrische Feld auf einen möglichst kleinen Bereich zu konzentrieren, um so eine möglichst starke Fokussierung zu erhalten.

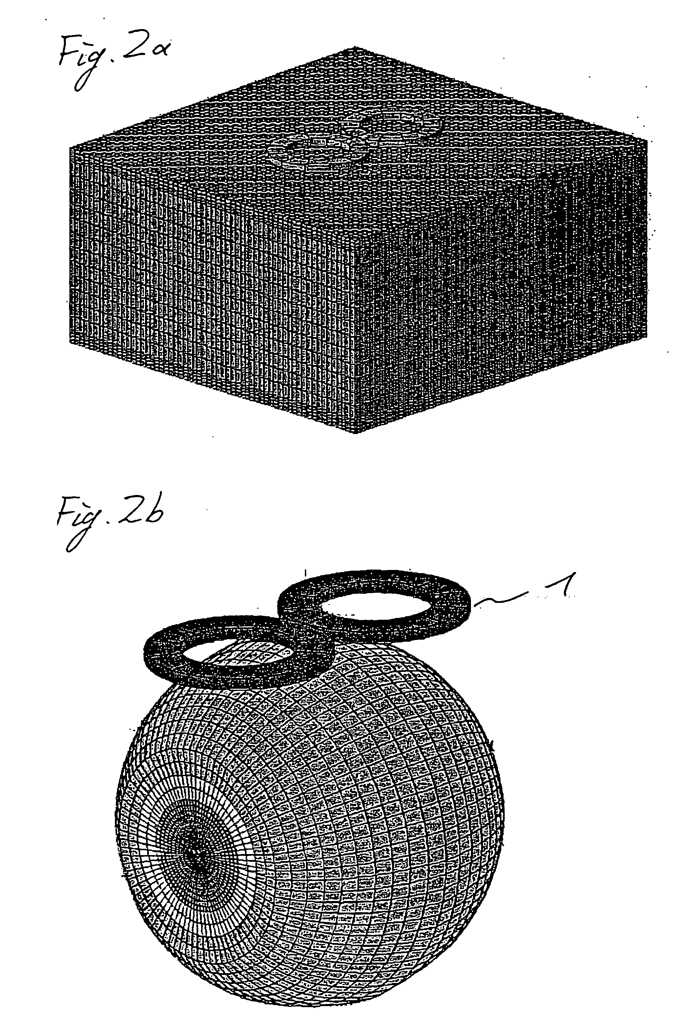

Fig. 2a zeigt schematisch ein Modell, welches bisher zur Modellierung eines elektrischen Feldes verwendet wurde, das durch zwei in einer Ebene nebeneinanderliegende Spulen, welche die Induktionsvorrichtung 1 bilden, erzeugt wurde. Dabei wurde jedoch nicht berücksichtigt, dass aufgrund der Krümmung des Kopfes das durch die Induktionsvorrichtung 1 auf der Hirnoberfläche induzierte elektrische Feld durch gekrümmte Schalen mit unterschiedlicher Leitfähigkeit und Dielektrizitätskonstante beeinflusst wird.

Fig. 2b zeigt schematisch die äußere Schale I des erfindungsgemäßen Drei-Schalen-Modells mit darüber liegender Induktionsvorrichtung 1, wodurch eine genauere Modellierung eines Kopfes und dadurch die genaue Fokussierung eines induzierten elektrischen Feldes ermöglicht wird.

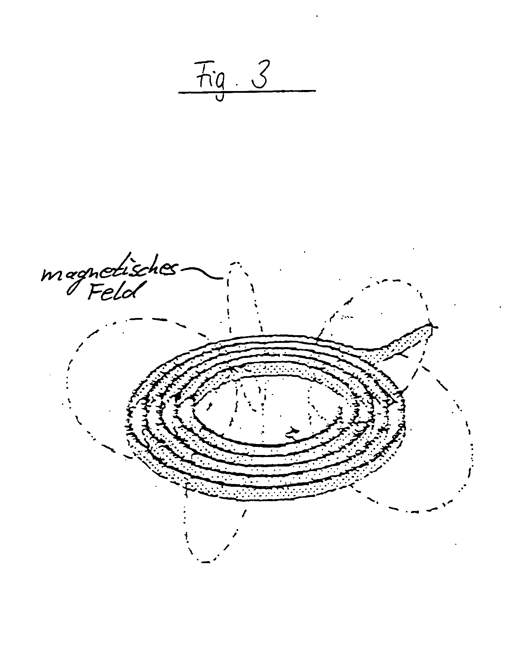

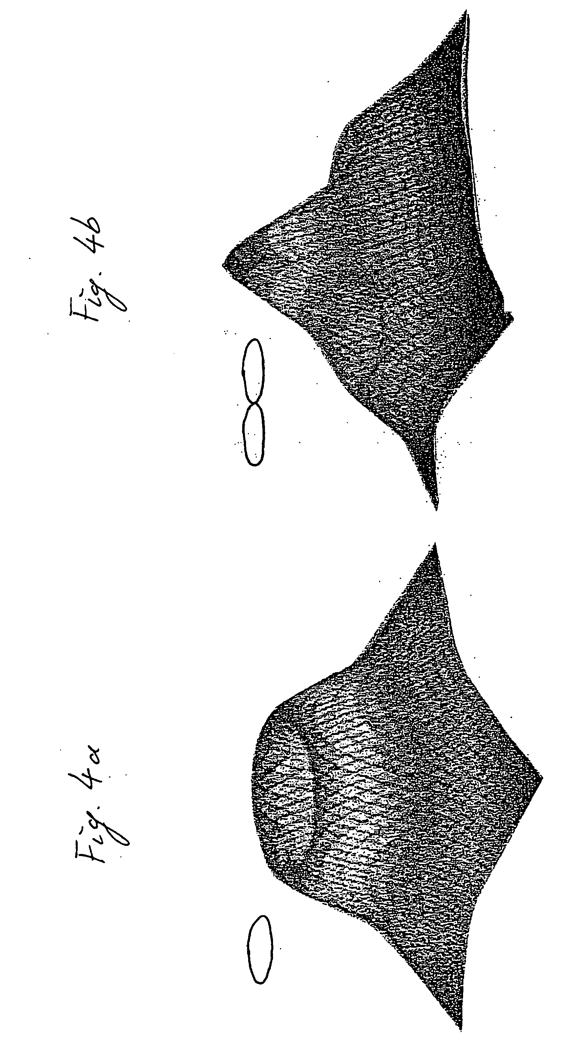

Fig. 3 zeigt schematisch eine mehrere Wicklungen aufweisende Spule, welche zum Beispiel als ein Teilelement der in Fig. 2b gezeigten Induktionsvorrichtung 1 verwendet werden kann. Wie aus Fig. 3 ersichtlich, ist die Spule nicht durch konzentrisch ineinander liegende Leiter gebildet, sondern durch ein einzelnes spiralförmig aufgewickeltes Leiterelement, wodurch eine leichte Asymmetrie des durch ein solches spiralförmiges Leiterelement erzeugten magnetischen Feldes vorliegt. Somit muss eine Untersuchung der konkret verwendeten Induktionsvorrichtung durchgeführt werden, um ein gutes Simulationsmodell zu erhalten. Die Annahme, dass zum Beispiel bei einer 8-förmigen Spule das Maximum des erzeugten Magnetfeldes unterhalb des Schnittpunktes liegt, trifft aufgrund der Asymmetrie einer Spulenwicklung im Allgemeinen nicht zu und führt zu schlechten Ergebnissen bei der TMS. Ein durch zum Beispiel zwei spiralförmig gewickelte nebeneinanderliegende Spulen erzeugtes magnetisches Feld weist die schematisch in Fig. 4b gezeigte magnetische Feldstärkeverteilung auf. Fig. 4a zeigt im Vergleich dazu das durch nur eine Spule erzeugte Feld. Es kann leicht erkannt werden, dass eine bessere Fokussierung mit hoher Feldstärke vorteilhaft durch eine Doppelspulenanordnung realisiert werden kann. Die in Fig. 4a gezeigte durch eine einzelne Spule erzeugte magnetische Feldstärkeverteilung ist auf Grund des ringförmigen Maximums nicht so gut auf einen kleinen Bereich fokussierbar, wie das durch eine Doppelspule erzeugbare magnetische Feld von Fig. 4b mit deutlichem spitz zulaufendem Maximum.

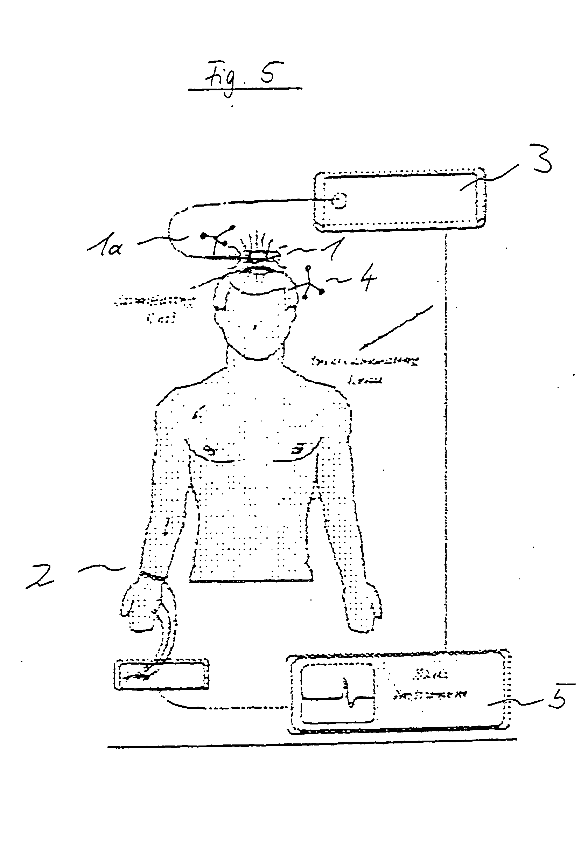

Fig. 5 zeigt schematisch eine erfindungsgemäße Vorrichtung zur Stimulation bestimmter Bereiche eines Gehirns, wobei eine mit einem Referenzstern 1a verbundene Induktionsvorrichtung 1 mit einer Ansteuervorrichtung 3 verbunden ist, welche Impulse erzeugt, um im Gehirn der gezeigten Person, an welcher ortsfest ein weiterer Referenzstern 4 angebracht ist, einen bestimmten Bereich zu stimulieren. Wird beispielsweise ein motorisches Hirnareal stimuliert, so kann mittels einer Oberflächenelektrode 2, welche zum Beispiel an dem Arm der Person angebracht ist, durch eine Elektromyographie (EMG)-Messung ein Anregungsimpuls gemessen werden, wie beispielhaft auf der Messvorrichtung 5 dargestellt. Durch eine Rückkopplung von der Messvorrichtung 5 zur Ansteuervorrichtung 4 kann automatisch eine möglichst genaue Lokalisation des motorischen Areals erfolgen, wobei beispielsweise die Induktionsvorrichtung 1 automatisch mittels eines Roboterarms in für eine oder mehrere Stimulationen geeignete Position gebracht wird.

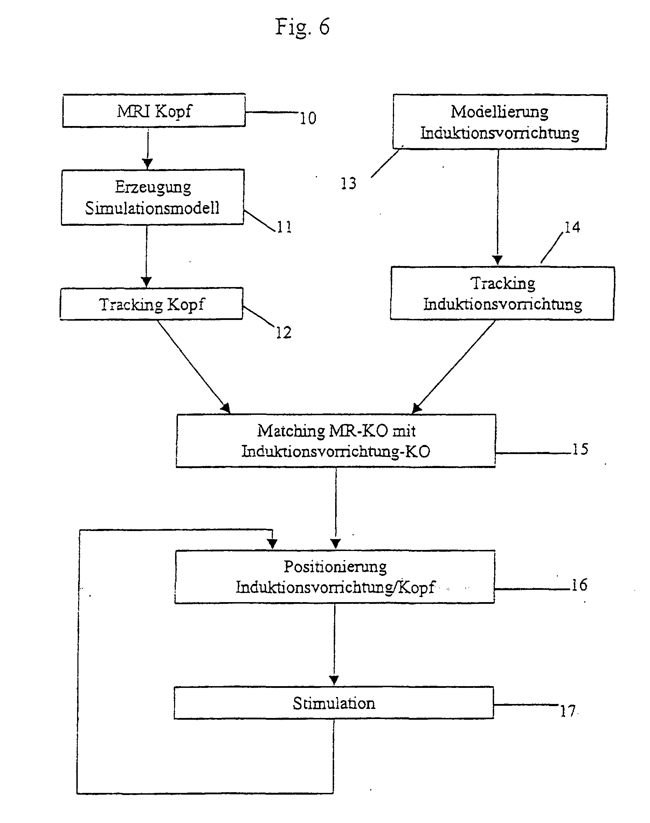

Fig. 6 zeigt schematisch das Ablaufdiagramm einer Ausführungsform des erfindungsgemäßen Verfahrens. In einem ersten Schritt 10 wird durch Kernspinresonanz (MRI) die räumliche Struktur eines Kopfes mit Gehirn aufgenommen. Die in Schritt 10 gewonnen Daten werden in Schritt 11 verwendet, um ein Simulationsmodell des aufgenommenen Kopfes zu erzeugen, wobei zum Beispiel wie oben beschrieben die Kopfhaut, der Schädelknochen und das Gehirn als drei Bereiche I, II und III modelliert werden, welche jeweils eine charakteristische Dielektrizitätskonstante und einen charakteristischen Leitwert aufweisen. Der Kopf ist mit einem Referenzstern 4 verbunden, wie in Fig. 5 gezeigt, wodurch dessen räumliche Position jederzeit einfach erfasst werden kann (Tracking).

In Schritt 13 wird die verwendete Induktionsvorrichtung 1 modelliert, wobei zur Modellierung Daten verwendet werden können, welche aus einer genauen Untersuchung der räumlichen Struktur von in der Induktionsvorrichtung enthaltenen Leitern bzw. Spulen gewonnen wurden, so dass ein durch die Induktionsvorrichtung erzeugbares magnetisches Feld relativ genau berechnet und simuliert werden kann. Weiterhin kann die Induktionsvorrichtung auch durch Auswertung von Messungen in dem durch die Induktionsvorrichtung erzeugten magnetischen Feld modelliert werden. Durch die Modellierung kann ein Fokussierbereich einer konkret verwendeten Induktionsvorrichtung relativ genau bestimmt werden. Die Induktionsvorrichtung wird, wie in Fig. 5 gezeigt, mit einem Referenzstern 1a verbunden, wodurch die Induktionsvorrichtung 1 ebenso wie der mit einem Referenzstern 4 verbundene Kopf getrackt werden kann.

Es wird in Schritt 15 ein Abgleich (Matching) der Koordinaten des Kopfes und damit der räumlichen Lage der durch MRI gewonnenen Struktur des Gehirns mit den Koordinaten der Induktionsvorrichtung vorgenommen, wodurch die relative räumliche Lage der Induktionsvorrichtung zu der durch die Kernspintomographie ermittelten räumlichen Struktur des Kopfes, insbesondere des Gehirns erhalten werden kann. Unter Verwendung dieser nun bekannten räumlichen Lagebeziehung kann in Schritt 16 eine Positionierung der Induktionsvorrichtung am Kopf erfolgen, wobei die Modellierungsdaten der Induktionsvorrichtung und die Modellierungsdaten des Kopfes verwendet werden, um den bei einem Stromfluss durch die Induktionsvorrichtung erzeugten Induktionsbereich auf dem Gehirn zu simulieren. Ist die Positionierung so durchgeführt worden, dass in der Simulation ein möglichst kleiner Bereich des Gehirns durch die Induktionsvorrichtung stimuliert wird, so wird in Schritt 17 die Stimulation dieses durch eine Simulation vorab ermittelten Bereiches durchgeführt. Ein Beobachter kann nun feststellen, was eine Person für spezifische Reaktionen zeigt, wenn dieser bestimmte Bereich stimuliert wurde, wobei anhand dieser Reaktionen, wie zum Beispiel dem Zucken eines Muskels, Störung des Sprachverhaltens oder ähnliches ermittelt werden kann, ob der stimulierte Bereich des Gehirns eine bestimmte Funktionalität besitzt. Wird dieser Vorgang der Positionierung und Stimulation bei einer Vielzahl von Bereichen des Gehirns durch Wiederholen der Schritte 16 und 17 durchgeführt, so kann eine Kartierung von Hirnfunktionen vorgenommen werden, wobei insbesondere die primären Hirnareale der untersuchten Person lokalisiert werden können. Dabei muss das gewünschte induzierte elektrische Feld unter Verwendung der Simulationsmodelle wieder neu berechnet werden, wobei zum Beispiel bezüglich der Induktionsvorrichtung bis zu sieben Freiheitsgrade (drei für Translation, drei für Rotation und einer für Spulenstrom) zu berücksichtigen sind.

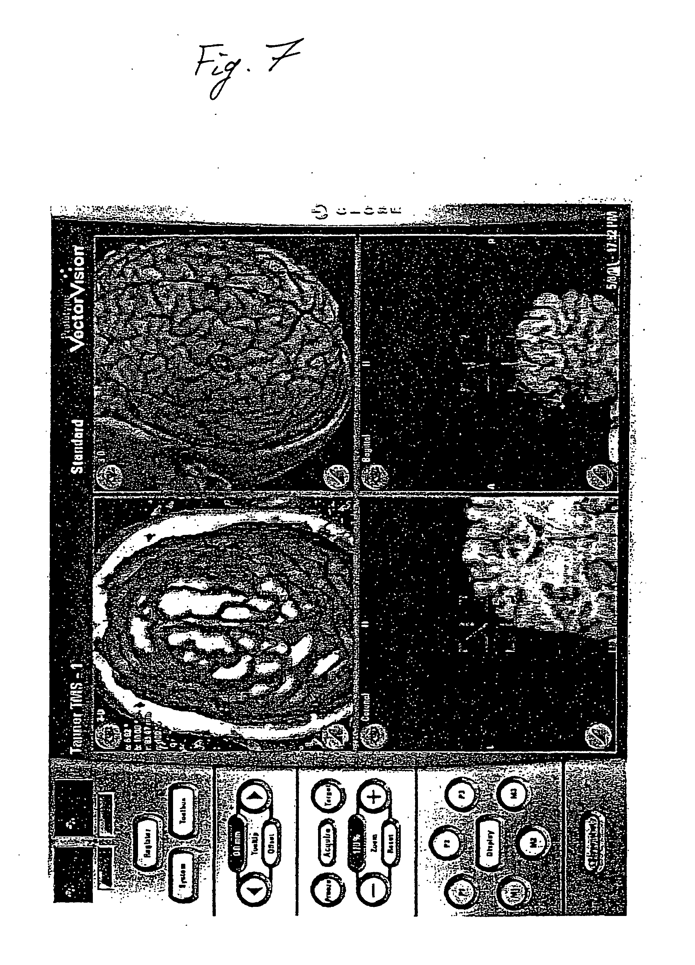

Fig. 7 zeigt in den oberen beiden Bildhälften die Struktur eines Gehirns basierend auf durch Kernspinresonanz gewonnene Daten aus zwei verschiedenen Blickwinkeln. Die darunter liegenden beiden Bilder zeigen die relative Lage der Induktionsvorrichtung zum Gehirn, welche in Abhängigkeit von angezeigten Steuersignalen in eine zur Stimulation geeignete Position gebracht werden kann.

## Patentansprüche

1. Vorrichtung zur Stimulation bestimmter Bereiche eines Gehirns mit einer Induktionsvorrichtung (1), welche mit mindestens einem Marker (1a) verbunden ist und mit mindestens einem fest mit dem Kopf verbundenen Marker (4), einer Positionserfassungsvorrichtung zur Erfassung der Position der Marker (1a, 4) und einer Simulationsvorrichtung zum Bestimmen des durch die Induktionsvorrichtung (1) stimulierbaren Stimulationsbereiches im Gehirn, **dadurch gekennzeichnet dass** zur Simulation ein Modell der Induktionsvorrichtung (1) verwendet wird.

2. Vorrichtung nach Anspruch 1, wobei eine Anzeigevorrichtung zur Anzeige des durch die Induktionsvorrichtung (1) stimulierbaren Bereiches auf dem Gehirn vorgesehen ist.

3. Vorrichtung nach einem der Ansprüche 1 oder 2, wobei eine Vorrichtung zur automatischen Positionierung der Induktionsvorrichtung (1), insbesondere ein Roboterschwenkarm vorgesehen ist.

## Claims

1. A device for stimulating specific areas of a brain using an induction device (1) connected to at least one marker (1a) and comprising at least one marker (4) connected securely to the head, a position detection device for detecting the position of the markers (1a, 4) and a simulation device for determining the area of stimulation in the brain which may be stimulated by the induction device (1), **characterised in that** a model of the induction device (1) is used for simulating.

2. The device as set forth in claim 1, wherein a display device is provided for displaying the area on the brain which may be stimulated by the induction device (1).

3. The device as set forth in any one of claims 1 or 2, wherein a device is provided for automatically positioning the induction device (1), in particular a rotating robot arm.

## Revendications

1. Dispositif de stimulation de zones déterminées d'un cerveau avec un dispositif d'induction (1), lequel est relié à au moins un marqueur (1a) et au moins un marqueur (4) relié de manière fixe à la tête, un dispositif d'acquisition de position pour l'acquisition de la position des marqueurs (1a, 4) et un dispositif de simulation pour la détermination de la zone de stimulation dans le cerveau pouvant être stimulée par le dispositif d'induction (1), **caractérisé en ce qu'**on utilise pour la simulation un modèle du dispositif d'induction (1).

2. Dispositif suivant la revendication 1, un dispositif d'affichage étant prévu pour l'affichage de la zone pouvant être stimulée sur le cerveau par le dispositif d'induction (1).

3. Dispositif suivant l'une des revendications 1 ou 2, un dispositif de positionnement automatique du dispositif d'induction (1), en particulier un bras pivotant de robot, étant prévu.
